# EUROPÄISCHE PATENTSCHRIFT

(11) **EP 2 393 504 B1**
(45) Veröffentlichungstag und Bekanntmachung des Hinweises auf die Patenterteilung: **05.06.2013**
(21) Anmeldenummer: 10716268.7
(22) Anmeldetag: 08.02.2010
(51) Int. Cl.: A61K 38/00

(54) **EIN L-RIBOZYM ENTHALTENDE PHARMAZEUTISCHE ZUSAMMENSETZUNG ZUR BEHANDLUNG VON NEBENWIRKUNGEN DURCH GABE VON SPIEGELMEREN**
PHARMACEUTICAL COMPOSITIONS COMPRISING AN L-RIBOZYME FOR TREATING SIDE EFFECTS CAUSED BY SPIEGELMERS
COMPOSITIONS PHARMACEUTIQUES RENFERMANT UN RIBOZYME POUR LE TRAITEMENT DES EFFETS SECONDAIRES DE SPIEGELMERS

(30) Priorität: 06.02.2009 DE 102009007929; 12.08.2009 DE 102009036965
(43) Veröffentlichungstag der Anmeldung: 14.12.2011
(73) Patentinhaber: Freie Universität Berlin, 14195 Berlin (DE)
(72) Erfinder: ERDMANN, Volker, 14163 Berlin (DE); WYSZKO, Eliza, PL-61-465 Poznan (PL)
(74) Vertreter: Jungblut, Bernhard Jakob
(86) Internationale Anmeldenummer: PCT/DE2010/000159
(87) Internationale Veröffentlichungsnummer: WO 2010/088899

(56) Entgegenhaltungen:
- US-A1- 2003 219 422
- MAEDA A ET AL: "INHIBITION OF VIRAL MULTIPLICATION IN ACUTE AND CHRONIC STAGES OF INFECTION BY RIBOZYMES TARGETD AGAINST THE POLYMERASE GENE OF MOUSE HEPATITIS VIRUS" ADVANCES IN EXPERIMENTAL MEDICINE AND BIOLOGY, SPRINGER, US, Bd. 380, 1. Januar 1995 (1995-01-01), Seiten 399-404, XP008023685 ISSN: 0065-2598
- VATER A ET AL: "TOWARD THIRD-GENERATION APTAMERS: SPIEGELMERS AND THEIR THERAPEUTIC PROSPECTS" CURRENT OPINION IN DRUG DISCOVERY AND DEVELOPMENT, CURRENT DRUGS, LONDON, GB, Bd. 6, Nr. 2, 1. Januar 2003 (2003-01-01), Seiten 253-261, XP008031617 ISSN: 1367-6733 in der Anmeldung erwähnt

## Beschreibung

### Technisches Feld der Erfindung

Die Erfindung betrifft die Verwendung eines L-Ribozymes zur Herstellung einer pharmazeutischen Zusammensetzung, eine pharmazeutische Zusammensetzung enthaltend ein solches L-Ribozym sowie ein Verfahren zur Herstellung einer solchen pharmazeutischen Zusammensetzung.

### Hintergrund der Erfindung und Stand der Technik

Aptamere sind meist doppelsträngige D-Nukleinsäuren, welche spezifisch an ein beliebiges Targetmolekül binden, analog einer Antikörper/Antigen-Reaktion (Ellington, A.D. et al., Nature 346:818-822 (1990)). Für ein vorgegebenes Targetmolekül spezifische Aptamere werden beispielsweise durch das SELEX-Verfahren aus Nukleinsäurebibliotheken isoliert (Tuerk, C. et al., Science 249:505-510 (1990)).

Aptamere haben im therapeutischen Bereich den Zweck, u.a. unerwünschte Stoffwechselprodukte zu binden und dadurch zu inhibieren. Lediglich beispielsweise seien hierfür onkogene Genprodukte genannt. Bei der therapeutischen Anwendung von Aptameren nachteilig ist, dass diese eine unvorteilhafte Pharmakokinetik aufweisen, i.e. sehr schnell abgebaut werden, beispielsweise durch endogene Nukleasen. Unabhängig hiervon sind Aptamere ohnehin relativ kleine Moleküle, welche daher vergleichsweise schnell über die Niere ausgeschieden werden.

Spiegelmere sind im Kern Aptamere, unterscheiden sich davon jedoch dadurch, dass sie aus L-Nukleotiden gebildet werden. Spiegelmere können einzel- oder doppelstängig sein. Durch den Einsatz der L-Nukleotiden wird ein Abbau durch endogene Nukleasen verhindert und so die Pharmakokinetik erheblich verbessert, i.e. die Verweilzeit im Serum verlängert. So ist in der Literaturstelle Boisgard, R et al., Eur Journal of Nuclear Medicine and Molecular Imaging 32:470-477 (2005) beschrieben, dass nicht-funktionelle Spiegelmere metabolisch auch über einen Zeitraum von 2 Stunden völlig stabil sind. In dieser Literaturstelle ist auch der diagnostische Einsatz von Spiegelmeren beschrieben, wobei das Spiegelmer mit einer beispielsweise radioaktiven Reportersubstanz gekoppelt ist.

Die Identifizierung von für ein vorgegebenes Targetmolekül spezifischen Spiegelmeren kann beispielsweise wie in der Literaturstelle Klussmann, S. et al., Nat Biotechnol 14:1112-1115 (1996) beschrieben erfolgen. Zu den Spiegelmeren und deren therapeutische Anwendungsmöglichkeiten wird auch auf die Literaturstelle Vater, A. et al., Curr Opin Drug Discov Devel 6:253-261 (2003) verwiesen.

In der therapeutischen Anwendung von Spiegelmeren ist bislang davon ausgegangen worden, dass Spiegelmere nicht immunogen sind (Wlotzka et al., Proc Natl Acad Sci USA 99:8898-8902 (2002)). Allerdings zeigen Untersuchungen, die in der vorliegenden Beschreibung präsentiert werden, dass L-Nukleinsäuren in einem Organismus durchaus nicht **notwendigerweise** nebenwirkungsfrei sind. Hieraus folgt, dass bei dem Einsatz von Spiegelmeren durchaus ein nicht vernachlässigbares Risiko einer unerwünschten physiologischen Nebenreaktion, beispielsweise einer Immunreaktion und/oder einer unerwünschten enzymatischen Reaktion mit endogener RNA (einschließlich einer regulatorischen RNA), bei Verabreichung an einen Patienten besteht. Insbesondere im Lichte der in der klinischen Phase 1 gemachten negativen Erfahrungen mit dem monoklonalen Antikörper TGN1412 und vor dem Hintergrund, dass die Verweilzeit von Spiegelmeren aufgrund der vorstehend genannten Zusammenhänge vergleichsweise sehr hoch ist, wäre es wünschenswert, ein Antidot für ein einzusetzendes Spiegelmer bei der Gabe des Spiegelmers bereit zu halten, damit bei Auftreten einer unerwünschten physiologischen Nebenreaktion unverzüglich das Antidot verabreicht und so der Spiegelmer-Pegel im Serum kurzfristig gesenkt werden kann.

Aus anderen Zusammenhängen, nämlich der Ribozymkatalysierten stereoselektiven Diels-Alder Reaktion sind L-Ribozyme bekannt, wozu auf die Literaturstellen Seelig, B. et al., Angew.Chem. Int., 39:4576-4579 (2000) und Seelig, B. et al., Angew. Chem. 112:4764-4768 (2000) verwiesen wird.

### Technisches Problem der Erfindung.

Der Erfindung liegt daher das technische Problem zugrunde, ein Antidot für therapeutisch eingesetzte Spiegelmere anzugeben.

### Grundzüge der Erfindung

Zur Lösung dieses technischen Problems lehrt die Erfindung die Verwendung eines L-Ribozymes zur Herstellung einer pharmazeutischen Zusammensetzung, wobei das L-Ribozym zur Spaltung einer L-RNA im Bereich einer Targetsequenz der L-RNA befähigt ist, und zwar insbesondere zur Herstellung einer pharmazeutischen Zusammensetzung zur Behandlung von unerwünschten physiologischen Nebenreaktionen, insbesondere Immunreaktionen und/oder unerwünschten enzymatischen Reaktionen der L-RNA mit endogener RNA (einschließlich einer regulatorischen RNA), aufgrund der Gabe eines therapeutischen Moleküls enthaltend die L-RNA.

Die Erfindung beruht zunächst auf der überraschenden Erkenntnis, dass Spiegelmere entgegen bisheriger Vermutungen nicht notwendigerweise frei von Nebenreaktionen sind, sondern vielmehr in der Lage sein können, natürlicherweise in einem Organismus vorkommende Nukleinsäuren zu schneiden und so unvorhersehbare Nebenwirkungen zu erzeugen. Die Erfindung beruht auf dieser Erkenntnis aufbauend auf der technischen Lehre, L-Ribozyme zur, Verfügung zu stellen, welche spezifisch ein verabreichtes Spiegelmer schneiden und so deren physiologische Wirksamkeit, insbesondere in Hinblick auf unerwünschte Nebenreaktionen, zu zerstören. Beispiele für Spiegelmere sind: Spiegelmer, NOXC89, NOXA42, NOXA50, NOXB11, NOXA12, NOXE36, NOXF37 (alle NOXXON AG), Spiegelmere der Firma Eli Lilly & Co., NU172 de Firma ARCA biopharma Inc., ARCHEMIX, ARC1905, ARC1779, ARC183, ARC184, E10030, NU172, REG2, REG1 (alle Archemix Corp.), AS1411, AS1405 (beide Antisoma Research Ltd.), DsiRNA von Dicerna Pharmaceuticals Inc., RNA Aptamer BEXCORE der BexCore Inc., ELAN der Firma Elan Corp Plc., oder Macugen. Durch die Gabe eines solchen Ribozymes in Verfolg der Beobachtung einer unerwünschten Nebenreaktion bei Gabe eines Spiegelmeres kann folglich die Ursache der unerwünschten Nebenreaktion aus dem Stoffwechsel schnell, effektiv und hochselektiv entfernt werden, und zwar bei wiederum extrem niedrigen Risiko von Nebenwirkungen der Gabe des L-Ribozymes. Letzteres beruht nicht nur auf dem Aufbau des L-Ribozymes aus L-Nukleotiden, sondern zusätzlich auf der hohen Selektivität des L-Ribozymes, nämlich gerichtet auf die Targetsequenz des Spiegelmeres. Im Ergebnis wird ein hochwirksames und hochselektives Antidot gegen ein therapeutisch eingesetztes Spiegelmer erhalten und unerwünschten Nebenreaktionen des Spiegelmeres kann effektiv, schnell und nebenwirkungsfrei begegnet werden.

Grundsätzlich kann gegen jedes RNA Molekül, sei es aus D- oder L-Nukleotiden aufgebaut, ein spezifisches Ribozym konstruiert werden, welches eine Targetsequenz des RNA Moleküls schneidet und diese so spaltet. Eine wesentliche Eigenschaft eines Ribozymes ist also die sequenzspezifische Bindung des Ribozymes an die Targetsequenz. Dies bedeutet aber auch, dass für jede beliebige Targetsequenz eine Teilsequenz eines Ribozymes dadurch erstellt werden kann, dass die Teilsequenz des Ribozymes, enthaltend die Spaltungsstelle, mit der Targetsequenz hybridisiert. Daher ist es im Rahmen der Erfindung nicht zweckmäßig nur bestimmte Ribozym-Teilsequenzen in Bezug auf bestimmte Targetsequenzen strukturell anzugeben. Die in den Beispielen angegebenen Targetsequenzen und Ribozym-Teilsequenzen sind daher lediglich beispielhaft und der Fachmann kann ohne weiteres für jede vorgegeben Targetsequenz eines Spiegelmeres die passende, nämlich hybridisierende Ribozym-Teilsequenz bestimmen und das Ribozym auf der Basis der Informationen zur Ribozym-Teilsequenz mit fachüblichen Mitteln synthetisieren.

Grundsätzlich kann das therapeutische Molekül ein Spiegelmer sein, oder die L-RNA kann mit einem Aptamer covalent gebunden sein. Der letztere Fall kann beispielsweise im Falle eines gegen Nukleasen stabilisierten Aptamers vorliegen. Dann liegt der therapeutische Nutzen der Erfindung darin, dass durch das Schneiden der L-RNA das Aptamer für Nukleasen zugänglich gemacht wird, wodurch dann letztendlich die auch ein eventuell Nebenwirkungen verursachendes Aptamer aus dem Serum vergleichsweise kurzfristig eliminiert werden kann.

Es ist aber auch möglich, dass das L-Ribozym covalent mit einem Aptamer oder einem Antikörper gebunden ist. Dabei kann das Aptamer oder der Antikörper beispielsweise so ausgewählt sein, dass auf Grund der Wechselwirkungen des Aptamers bzw. des Antikörpers mit Zelloberflächen das Gesamtkonstrukt aus L-Ribozym und Aptamer bzw. Antikörper in die Zelle eingeführt wird.

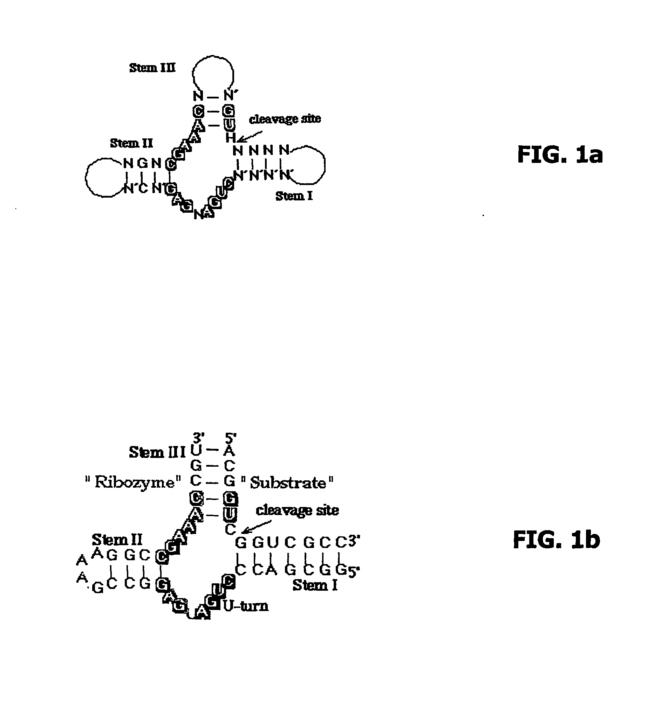

Vorzugsweise ist das L-Ribozym ein Hammerhead Ribozym. Hammerhead Ribozyme weisen eine konservierte Region u.a. mit einem Triplett GUH (H ist kein Guanin, vorzugsweise C) oder einem Duplet UH (H wie oben) auf. Zu ersterem wird auf die Figur 1 verwiesen. Zu letzterem wird auf die Literaturstelle Usman, N, et al., The Journal of Clinical Investigation, 106(10):1197-1201 (2000) verwiesen. Hierin sind die Nukleotide N' und N beliebige Basen, welche im Bereich der Stems I und III nach Maßgabe der Targetsequenz ausgewählt werden. Im Kern geht man bei der Konstruktion eines L-Ribozymes gegen eine Targetsequenz so vor, dass man zunächst eine Targetsequenz, beispielsweise eines Spiegelmers, vorgibt, wobei diese Targetsequenz das Triplet GUH oder das Duplet UH enthalten muss. Dann werden an beiden Enden eines Tripletts GUH bzw. des Duplets UH typischerweise jeweils 4-10 oder 4-11, insbesondere 6-8 oder 6-9 Nukleotide angefügt, deren Sequenzen den Sequenzen der Targetsequenz entspricht. Man erhält also eine 11 bis 23 Nukleotide enthaltende Kopie der Targetsequenz enthaltend das Triplett GUH bzw. das Duplet UH. Zwischen den beiden Enden der Kopie wird dann die katalytische Hammerhead-Sequenz, wie in der Figur 1 ersichtlich, eingefügt. Ein Beispiel für eine geeignete katalytische Hammerhead-Sequenz ist folglich:
5'-CUGANGAGN'CN'NNNNNGNCGAAAC-3' oder
5'-CUGANGAGN'CN'NNNNNGNCGAAAN-3'
(N = beliebige Basen, wobei in Figur 1 sich gegenüberstehende N und N' zwingend gleiche oder verschiedene Basenpaare bilden)

Hieran schließen sich am 3'-Ende Nukleotide in der Sequenz komplementär zur Targetsequenz 5'-seitig des Tripletts GUH bzw. Duplets UH und am 5'-Ende Nukleotide in der Sequenz entsprechend der Targetsequenz 3'-seitig des Tripletts GUH bzw. Duplets UH an.

In einer bevorzugten Ausführungsform lautet die katalytisch Hammerhead Sequenz
5'-CUGANGAGNUCGGAAACGACGAAAC-3' oder
5'-CUGANGAGNUCGGAAACGACGAAAN-3'
(N = beliebige Basen, wobei in Figur 1 sich gegenüberstehende N und N' zwingend gleiche oder verschiedene Basenpaare bilden)
auf. Zusätzlich kann am 5'-Ende der katalytischen Hammerhead-Sequenz die Sequenz
3'-(N)₄₋₆GGUAUAGAGUGCUGAAUCC-5'
eingerichtet sein, wodurch ein Hammerhead Ribozym erhalten wird, welches eine vergleichsweise geringe Mg-Ionen Konzentration benötigt.

Die pharmazeutische Zusammensetzung enthält das L-Ribozym in zumindest der Dosis, welche der Dosis der Gabe der L-RNA entspricht, vorzugsweise in einer Dosis enthält, welche dem 2- bis 10-fachen, bezogen auf die Molekülanzahl bzw. Mole, der Dosis der Gabe der L-RNA entspricht. Eine Überdosierung, im Vergleich zur Dosis der L-RNA, wird sich empfehlen, um sicher zu gehen, dass alle zu eliminierende L-RNA abreagiert wird. Die erfindungsgemäß vorgesehenen absoluten Dosen werden sich dabei streng in den angegebenen relativen Mengenverhältnissen an den vorgegebenen Dosen der L-RNA richten und können daher vom Fachmann in Kenntnis der vorgegebenen Dosen für die L-RNA leicht bestimmt und eingerichtet werden.

In einer bevorzugten Ausführungsform der Erfindung enthält die pharmazeutische Zusammensetzung zusätzlich eine Nukleinsäure, insbesondere ein 5- bis 20-mer, welches zur Aufschmelzung einer doppelsträngigen L-RNA im Bereich derer Targetsequenz befähigt ist. Hierbei handelt es sich um Sequenzen, welche mit Teilsequenzen die der Targetsequenz benachbart sind, hybridisieren. Dadurch wird erreicht, dass GUC-Regionen der L-RNA, die normalerweise aus sterischen Gründen nicht zugänglich sind aufgrund der Tertiärstruktur der L-RNA, für das L-Ribozym zugänglich gemacht werden.

Des Weiteren betrifft die Erfindung eine pharmazeutische Zusammensetzung enthaltend ein L-Ribozym zur Behandlung von unerwünschten physiologischen Nebenreaktionen, insbesondere Immunreaktionen, aufgrund der Gabe eines therapeutischen Moleküls enthaltend die L-RNA.

Bezüglich der pharmazeutischen Zusammensetzung gelten alle vorstehenden und nachfolgenden Ausführungen analog.

Schließlich betrifft die Erfindung ein Verfahren zur Herstellung einer solchen pharmazeutischen Zusammensetzung, wobei eine Sequenz aus L-Nukleotiden erstellt und synthetisiert wird, welche zur Spaltung einer vorgegebenen Sequenz aus L-Ribonukleotiden, insbesondere enthaltend das Triplett GUC mit ansonsten beliebigen 5'- und 3'-seitig des Tripletts angefügten Sequenzen, befähigt ist, und wobei das L-Ribozym in pharmakologisch wirksamer Dosis zur Darreichung hergerichtet wird. Dabei wird das L-Ribozym typischerweise mit galenischen Hilfs- und/oder Trägerstoffen gemischt.

Grundsätzlich können ein oder mehrere physiologisch verträgliche Hilfstoffe und/oder Trägerstoffe mit dem L-Ribozym gemischt und die Mischung galenisch zur lokalen oder systemischen Gabe, insbesondere oral, parenteral, zur Infusion bzw. Infundierung in ein Zielorgan, zur Injektion (z.B. i.v., i.m., intrakapsulär oder intralumbal), zur Applikation in Zahntaschen (Raum zwischen Zahnwurzel und Zahnfleisch) und/oder zur Inhalation hergerichtet ist. Die Auswahl der Zusatz- und/oder Hilfsstoffe wird von der gewählten Darreichungsform abhängen. Die galenische Herrichtung der erfindungsgemäßen pharmazeutischen Zusammensetzung kann dabei in fachüblicher Weise erfolgen. Als Gegenionen für ionische Verbindungen kommen beispielsweise Mg⁺⁺, Mn⁺⁺, Ca⁺⁺, CaCl⁺, Na⁺, K⁺, Li⁺ oder Cyclohexylammonium, bzw. Cl⁻, Br⁻, Acetat, Trifluoracetat, Propionat, Laktat, Oxalat, Malonat, Maleinat, Citrat, Benzoat, Salicylat, Putrecin, Cadaverin, Spermidin, Spermin, usw. in Frage. Geeignete feste oder flüssige galenische Zubereitungsformen sind beispielsweise Granulate, Pulver, Dragees, Tabletten, (Mikro-) Kapseln, Suppositorien, Sirupe, Säfte, Suspensionen, Emulsionen, Tropfen oder Lösungen zur Injektion (i.v., i.p., i.m., s.c.) oder Vernebelung (Aerosole), Zubereitungsformen zur Trockenpulverinhalation, transdermale Systeme, sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Auch ist es möglich, den Wirkstoff in vorzugsweise biologisch abbaubaren Nanokapseln zu verkapseln, beispielsweise zur Herstellung einer Zubereitung zur Inhalation. Als Hilfsstoffe seien beispielsweise Magnesiumcarbonat, Titandioxid, Laktose, Mannit und andere Zucker, Talkum, Milcheiweiß, Gelatine, Stärke, Zellulose und ihre Derivate, tierische und pflanzliche Öle wie Lebertran, Sonnenblumen-, Erdnuss- oder Sesamöl, Polyethylenglykole und Lösungsmittel, wie etwa steriles Wasser und ein- oder mehrwertige Alkohole, beispielsweise Glycerin, genannt. Eine erfindungsgemäße pharmazeutische Zusammensetzung ist dadurch herstellbar, dass mindestens ein erfindungsgemäß verwendete Substanzkombination in definierter Dosis mit einem pharmazeutisch geeigneten und physiologisch verträglichen Träger und ggf. weiteren geeigneten Wirk-, Zusatz- oder Hilfsstoffen mit definierter Dosis gemischt und zu der gewünschten Darreichungsform hergerichtet ist. Als Verdünnungsmittel kommen Polyglykole, Wasser und Pufferlösungen in Frage. Geeignete Puffersubstanzen sind beispielsweise N,N'-Dibenzylethylendiamin, Diethanolamin, Ethylendiamin, N-Methylglucamin, N-Benzylphenethylamin, Diethylamin, Phosphat, Natriumbicarbonat, oder Natriumcarbonat. Es kann aber auch ohne Verdünnungsmittel gearbeitet werden. Physiologisch verträgliche Salze sind Salze mit anorganischen oder organischen Säuren, wie z.B. Milchsäure, Salzsäure, Schwefelsäure, Essigsäure, Citronensäure, p-Toluolsulfonsäure, oder mit anorganischen oder organischen Basen, wie z.B. NaOH, KOH, Mg(OH)₂, Diethanolamin, Ethylendiamin, oder mit Aminosäuren, wie Arginin, Lysin, Glutaminsäure usw. oder mit anorganischen Salzen, wie CaCl₂, NaCl oder deren freie Ionen, wie Ca²⁺, Na⁺, Cl⁻, SO₄²⁻ bzw. entsprechende Salze und freien Ionen des Mg⁺⁺ oder Mn⁺⁺, oder Kombinationen hieraus. Sie werden nach Standardmethoden hergestellt. Vorzugsweise wird ein pH-Wert im Bereich zwischen 5 und 9, insbesondere zwischen 6 und 8, eingestellt.

Von selbstständiger Bedeutung ist eine Variante der Erfindung, welche die Verwendung eines L-Ribozymes zur Herstellung einer pharmazeutischen Zusammensetzung zur Behandlung oder Prophylaxe von Erkrankungen, welche mit einer Überexpression zumindest eines endogenen Genes einhergehen, wobei das L-Ribozym zur Spaltung einer Targetsequenz einer für das Gen codierenden endogenen Target-D-RNA befähigt ist, umfasst. Ansonsten gelten die vorstehenden Ausführungen analog. In diesem Zusammenhang von Bedeutung ist eine weitere Variante des vorstehenden Aspekts der Erfindung mit der Verwendung eines L-Ribozymes zur Herstellung einer pharmazeutischen Zusammensetzung zur Behandlung oder Prophylaxe von Erkrankungen, welche mit einer Infektion eines Säugetiers mit einem Mikroorganismus einhergehen, wobei das L-Ribozym zur Spaltung einer Targetsequenz einer für ein Gen des Mikroorganismus codierenden Target-D-RNA befähigt ist. Als in Frage kommende Mikroorganismen sind u.a. Viren, Bakterien und Pilze zu nennen. Grundsätzlich kann das Ribozym zur Spaltung eines beliebigen Mikroorganismus mit zumindest teilweise bekannten Gensequenzen eingesetzt werden, wobei Bereiche der Gensequenzen zwecks Spaltung ausgewählt werden, welche beispielsweise die Aktivität des Mikroorganimus und/oder seine Replikationsfähigkeit attenuieren oder inhibieren und/oder die Bindung an Zelloberflächen attenuieren oder inhibieren.

In dieser Variante wird genutzt, dass L-Ribozyme auch zur Spaltung von D-RNA, insbesondere mRNA oder regulatorischer RNA, beispielsweise, aber nicht abschließend, siRNA, microRNA, shRNA, ncRNA, tRNA, rRNA, etc., eingesetzt werden können. Dadurch können Gene bzw. hierdurch codierte Proteine inhibiert werden. Dies ist von therapeutischem Nutzen für alle Erkrankungen, die mit der Überexpression bestimmter Gene, verglichen mit der Expression des nicht erkrankten Organismus, einhergehen.

Diese Variante hat zum einen den Vorteil, dass die Spaltung der Targetsequenz mit sehr hoher Spezifität erfolgt und somit auch keine sonstige Interferenz mit dem regulatorischen System erfolgt. Zudem werden Nebenwirkungen, wie sie beispielsweise mit dem Einsatz inhibitorischer D-Nukleinsäuren, wie siRNA, einhergehen, sicher vermieden werden.

Im folgenden wird die Erfindung anhand von Figuren und Beispielen näher erläutert.

Es zeigen:
- Figur 1:: ein Minimal-Hammerhead-Ribozym vor (a) und nach Bindung an eine Targetsequenz (b),
- Figur 2:: eine vergleichende Analyse der MgCl₂-Konzentrationsabhängigkeit der Reaktion von L-Target mit D-Ribozym einerseits und von D-Target mit L-Ribozym andererseits,
- Figur 3:: eine vergleichende Analyse der Zeitabhängigkeit der Reaktion von L-Target mit D-Ribozym einerseits und von D-Target mit L-Ribozym andererseits bei 10 mM MgCl₂,
- Figur 4:: eine vergleichende Analyse der MgCl₂-Konzentrationsabhängigkeit (1-25 mM) der Reaktion von L-Target mit L-Ribozym einerseits und von D-Target mit D-Ribozym andererseits bei 10-fachem L-Ribozym-Überschuss,
- Figur 5:: eine vergleichende Analyse der MgCl₂-Konzentrationsabhängigkeit (0,1-1 mM) der Reaktion von L-Target mit L-Ribozym einerseits und von D-Target mit D-Ribozym andererseits bei 10-fachem L-Ribozym-Überschuss,
- Figur 6:: eine vergleichende Analyse der Zeitabhängigkeit der Reaktion von L-Target mit L-Ribozym einerseits und von D-Target mit D-Ribozym andererseits bei 10 mM MgCl₂ und bei 10-fachem L-Ribozym-Überschuss,
- Figur 7:: eine vergleichende Analyse der Zeitabhängigkeit der Reaktion von L-Target mit L-Ribozym einerseits und von D-Target mit D-Ribozym andererseits bei 0,1 mM MgCl₂ und bei 10-fachem L-Ribozym-Überschuss,
- Figur 8:: eine vergleichende Analyse der Zeitabhängigkeit der Reaktion von L-Target mit L-Ribozym einerseits und von D-Target mit D-Ribozym andererseits bei 1 mM MgCl₂ und bei 1-fachem L-Ribozym-Überschuss,
- Figur 9:: eine vergleichende Analyse der Zeitabhängigkeit der Reaktion von L-Target mit L-Ribozym einerseits und von D-Target mit D-Ribozym andererseits bei 0,1 mM MgCl₂ und bei 10-fachem L-Ribozym-Unterschuss,
- Figur 10:: eine vergleichende Analyse der Zeitabhängigkeit der Reaktion von L-Target mit L-Ribozym einerseits und von D-Target mit D-Ribozym andererseits bei 1 mM MgCl₂ und bei 10-fachem L-Ribozym-Unterschuss,
- Figur 11:: eine vergleichende Analyse der Zeitabhängigkeit der Reaktion von L-Target mit L-Ribozym einerseits und von D-Target mit D-Ribozym andererseits bei 5 mM MgCl₂ und bei 10-fachem L-Ribozym-Unterschuss, und
- Figur 12:: Versuche zur Spaltung von L-Target durch L-Ribozym in humanem Serum.

### Beispiel 1: Cleavage assay

Die Aktivitäten von L-Ribozymen und D-Ribozymen wurden unter verschiedenen Bedingungen gemessen. Die Basisbedingungen waren wie folgt. 0,02 µM Target RNA wurden mit 10 µl Reaktionsmischung in der Gegenwart von 0,002 µM, 0,02µM und 2 µM Ribozym in 50 mM Tris-HCL Puffer, pH 7,5, bei 20°C für 2 Stunden inkubiert (Verhältnis Ribozyme/Target folglich 10:1, 1:1 und 1:10). Vor der Reaktion wurden Target RNA und Ribozym für 2 Minuten bei 70°C denaturiert und langsam (1°C/min.) in dem Heizblock auf 25°C abgekühlt. Es wurde der Einfluss der Mg²⁺-Ionen in Konzentration von 0,1 bis 25 mM untersucht. Spaltprodukte wurden auf 20% Polyacrylamid Gelelektrophorese in der Gegenwart von 8 M Harnstoff in 0,09 M Tris-Borat Puffer, pH 8,3, getrennt. Die Analyse der Fluoreszenz erfolgte auf Phosphoimager Fuji Film FLA 5100. Die Daten wurden mit dem Programm Fuji Analysis Program erhalten. Diagramme wurden mit Excel erstellt.

### Beispiel 2: Herstellung der Targetsequenzen und der Ribozyme

Als Targetsequenzen wurden im Wege der Auftragssynthese durch die Firma ChemGenes Corporation, Wilmington, USA, hergestellt:
Seq.-ID 1: 5'-FAM-ACAGUCGGUCGCC-3'
   (RNA, sowohl mit D-Nukleotiden, als auch mit L-Nukleotiden) und
Seq.-ID 2: 5'-FAM-ACAGTCGGTCGCC-3'
   (DNA, sowohl mit D-Nukleotiden, als auch mit L-Nukleotiden).

Die Syntheseprodukte hatten eine Reinheit von mehr als 90%

Als Ribozymsequenzen wurden nach Maßgabe der Targetsequenzen die variablen Bereiche eines Hammerhead Ribozyms um das Triplett GUC ausgewählt und die folgenden Ribozymesequenzen durch die Firma ChemGenes Corporation, Wilmington, USA, hergestellt:
Seq.-ID 3:5'-FAM-GGCGACCCUGAUGAGGCCGAAAGGCCGAAACUGU-3'
   (RNA, sowohl mit D-Nukleotiden, als auch mit L-Nukleotiden)

Die Syntheseprodukte hatten eine Reinheit von über 85%.

Alle Syntheseprodukte waren am 5'-Ende mit Fluoreszein markiert.

### Beispiel 3: Wechselwirkungen von L-Nukleinsäuren mit D-Nukleinsäuren

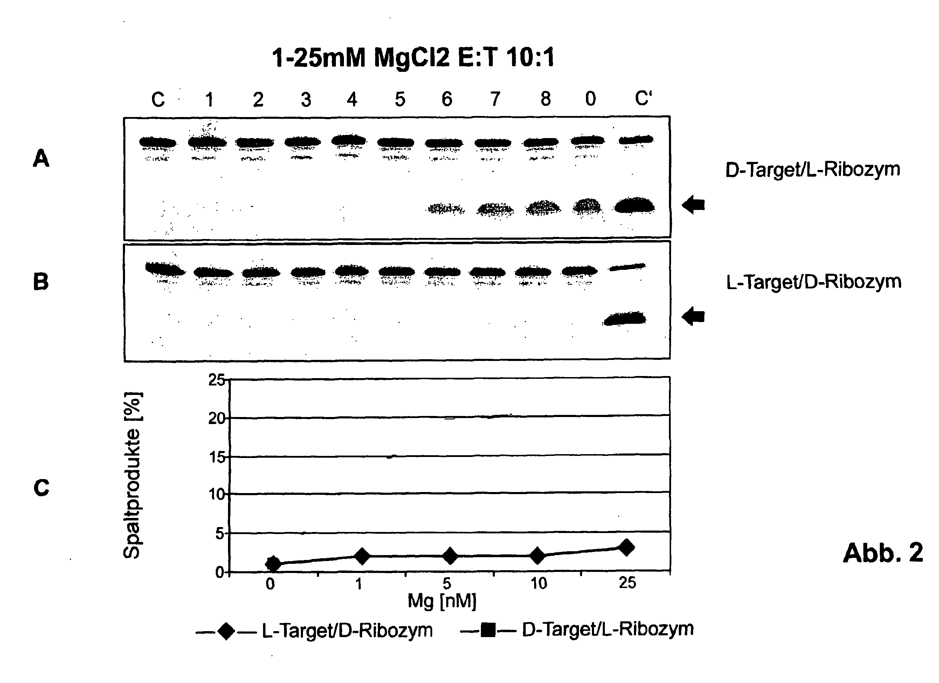

In der Figur 2 ist die Konzentrationsabhängigkeit der Spaltung eines D-Targets durch ein L-Ribozym sowie umgekehrt dargestellt. C ist die Kontrolle (L-Target + L-Ribozym), die Spuren 1 bis 5 sind die verschiedenen in dem Diagramm angegebenen MgCl₂ Konzentrationen (0 - 25 mM) für Target ohne Ribozym, die Spuren 6 bis 9 0,2 µM Target mit 2 µM Ribozym.

Man erkennt, dass D-Ribozym zwar nicht L-Target schneidet, aber umgekehrt durchaus ein beträchtlicher Umsatz stattfindet. Dies bedeutet, dass beispielsweise Spiegelmere, bestehend aus L-Nukleotiden, neben Ihrer Wirkung als für eine bestimmte 3-D-Struktur spezifisches Aptamer, entgegen bisherigen Auffassung durchaus in der Lage sein könnten weitere physiologische Wechselwirkungen auszuüben, beispielsweise als Ribozym.

Hieraus folgt, dass Spiegelmere die Gefahr einer unerwünschten Nebenwirkung bei Verabreichung an einen Organismus aufweisen.

Hieraus folgt aber auch, dass L-Ribozyme zur Spaltung von endogener D-RNA eingesetzt werden können, wodurch dann eine therapeutisch angestrebte Inhibierung des durch die D-RNA, beispielsweise mRNA, codierten Genes bzw. Proteins erfolgt.

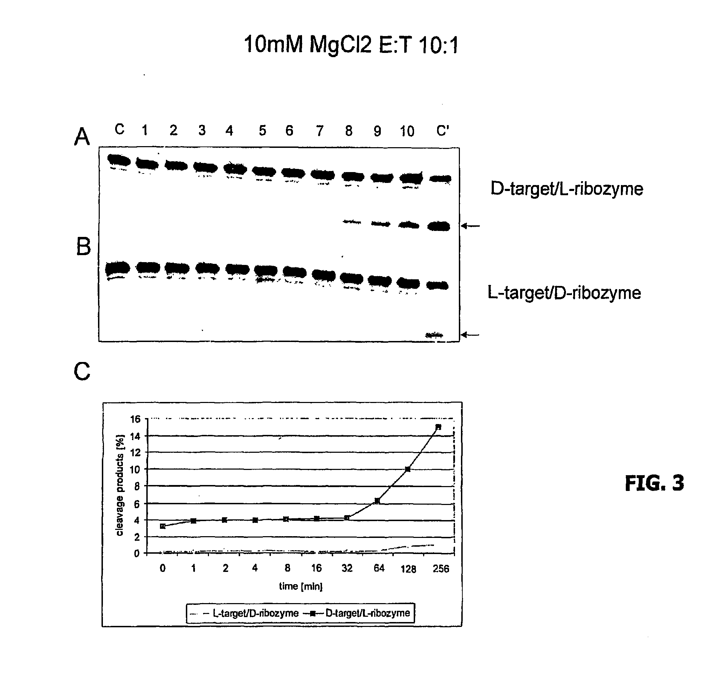

Die Figur 3 zeigt, dass der Anteil an Spaltprodukten des D-Targets durch ein L-Ribozym mit der Zeit anwächst und sich stets signifikant oberhalb des Anteils an Spaltprodukten des L-Targets beläuft (Spur C: Kontrolle, wie vorstehend, Spuren 1 bis 10 Zeiten 0 bis 256 min. des Diagramms).

### Beispiel 4: Spaltung eines L-Targets durch L-Ribozyme

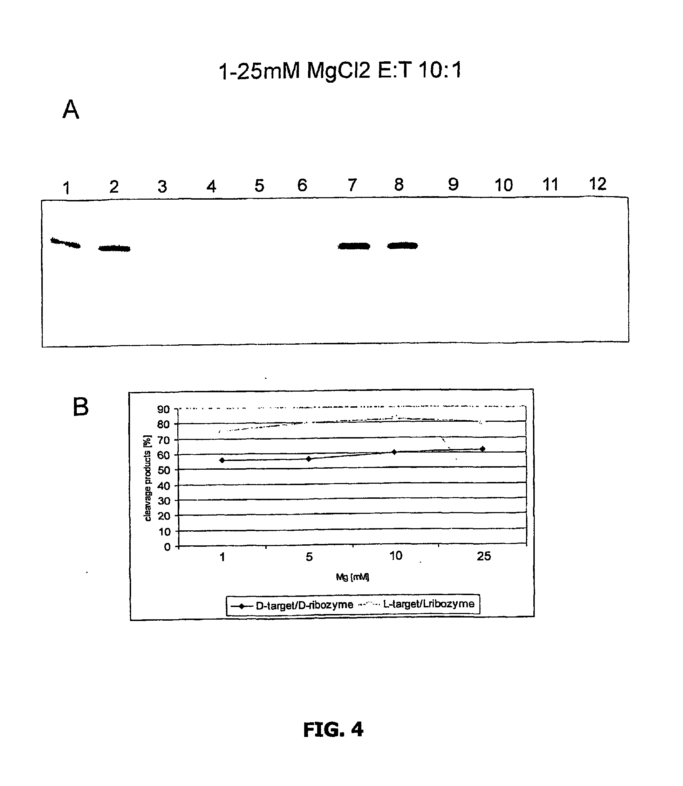

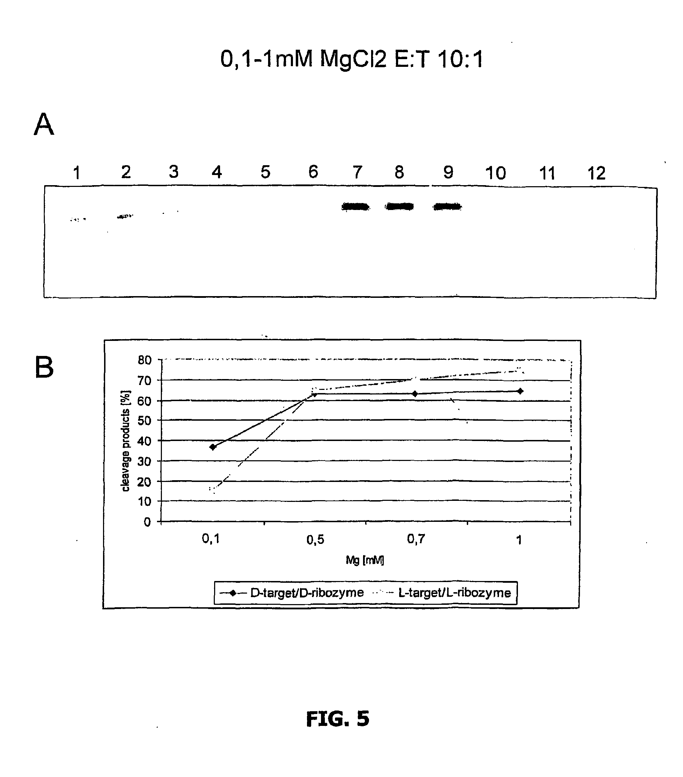

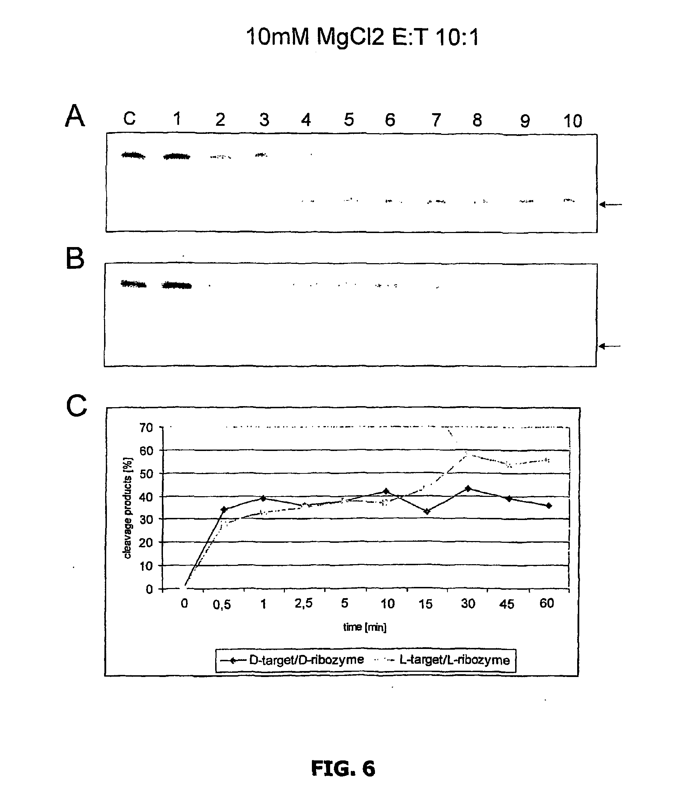

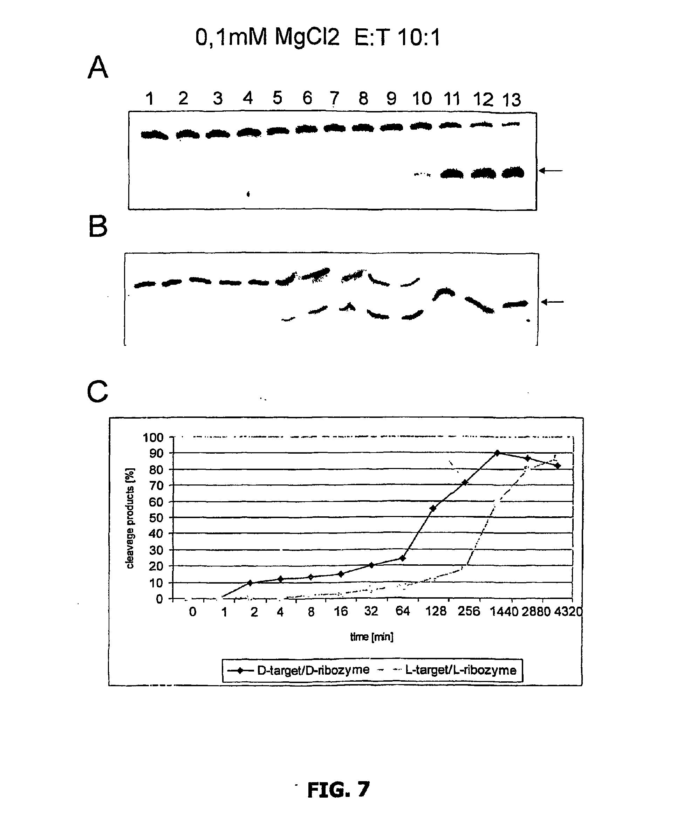

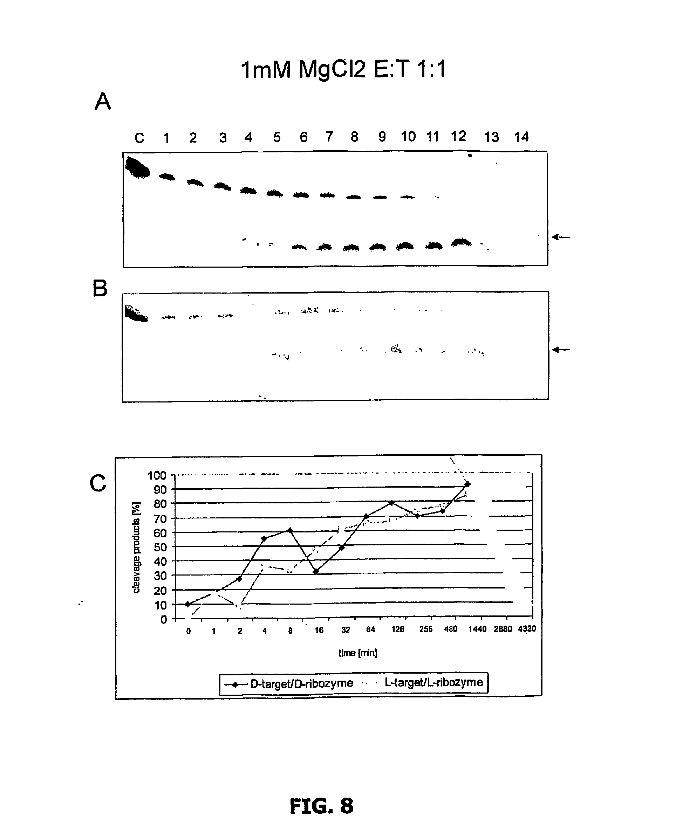

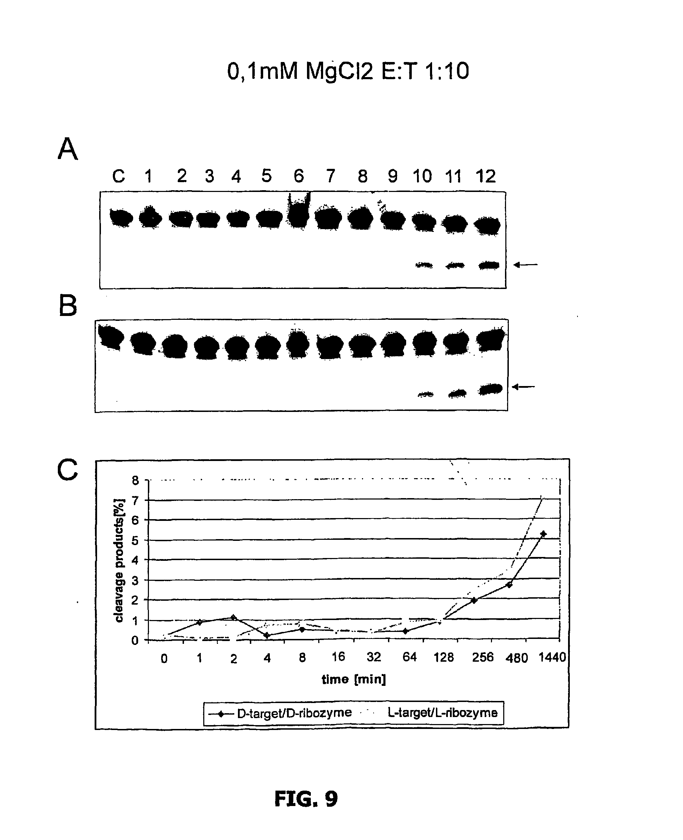

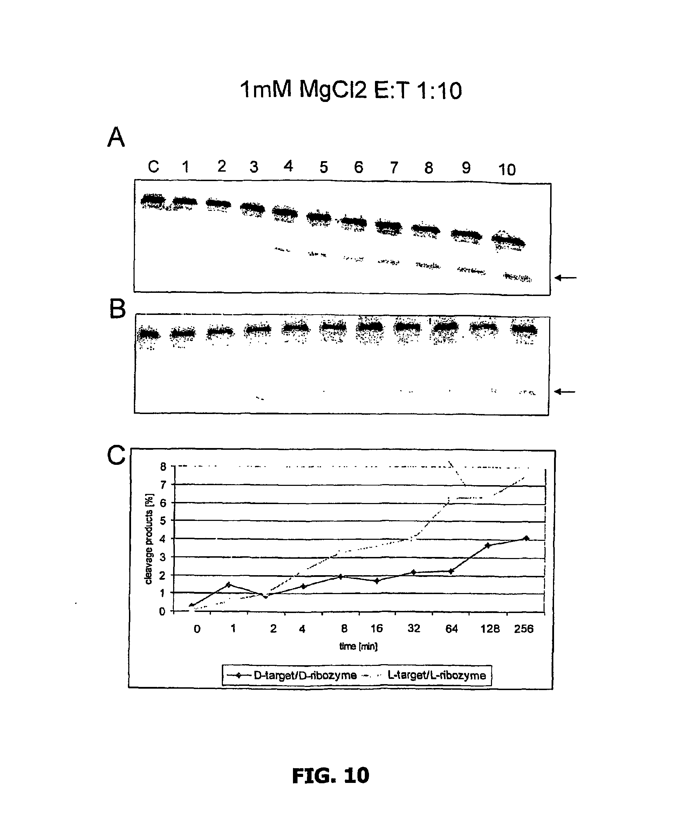

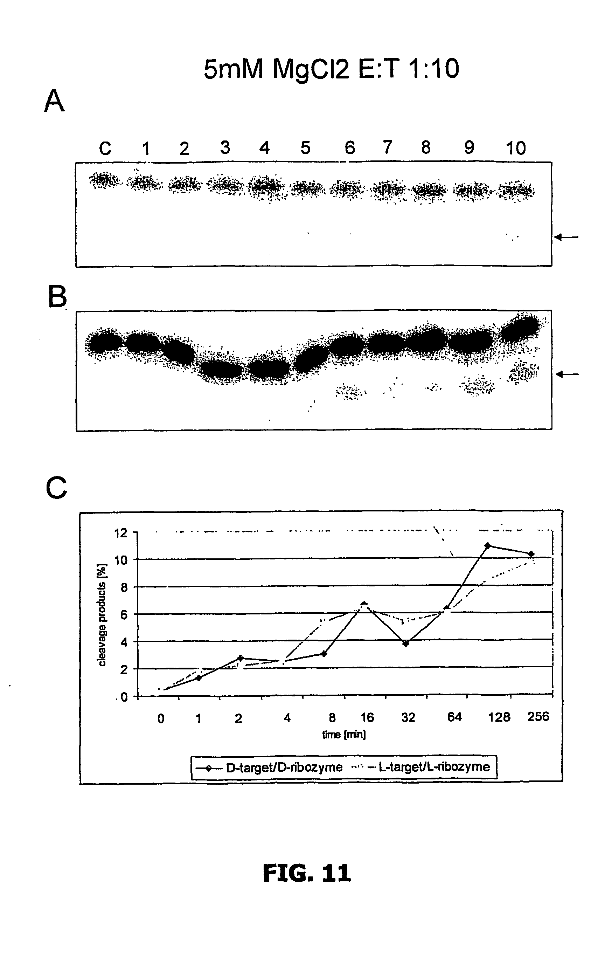

Den Darstellungen der Figuren 4 bis 11 ist zu entnehmen, dass ein L-Ribozym ein L-Target mit entsprechender Targetsequenz unter allen üblichen Bedingungen effektiv schneidet und zwar mit Umsatzraten, die jenen eines D-Ribozymes mit einem D-Target zumindest entsprechen.

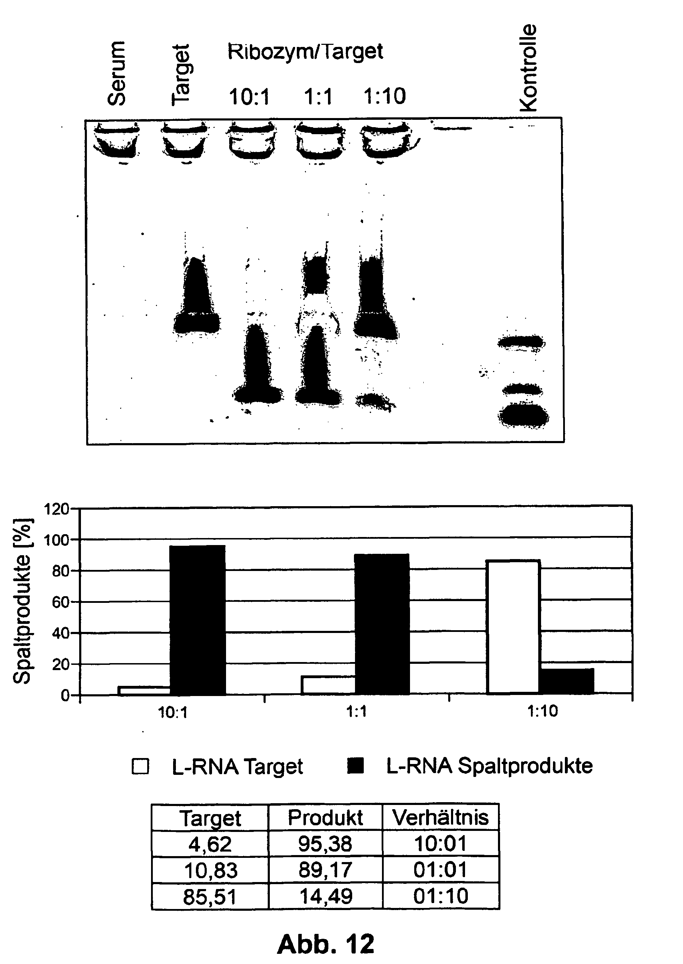

Die Figur 12 belegt, dass die Spaltung eines L-Targets durch ein L-Ribozym auch unter den Bedingungen des humanen Serums effektiv funktioniert.

## Patentansprüche

1. Verwendung eines L-Ribozymes zur Herstellung einer pharmazeutischen Zusammensetzung.

2. Verwendung nach Anspruch 1, wobei das L-Ribozym zur Spaltung einer L-RNA im Bereich einer Targetsequenz der L-RNA befähigt ist.

3. Verwendung eines L-Ribozymes, welches zur Spaltung einer L-RNA im Bereich einer Targetsequenz der L-RNA befähigt ist, zur Herstellung einer pharmazeutischen Zusammensetzung zur Behandlung von unerwünschten physiologischen Nebenreaktionen, aufgrund der Gabe eines therapeutischen Moleküls enthaltend die L-RNA,

4. Verwendung eines L-Ribozymes zur Herstellung einer pharmazeutischen Zusammensetzung zur Behandlung oder Prophylaxe von Erkrankungen, welche mit einer Überexpression zumindest eines endogenen Genes einhergehen, wobei das L-Ribozym zur Spaltung einer Targetsequenz einer für das Gen codierenden endogenen Target-D-RNA befähigt ist.

5. Verwendung nach Anspruch 3, wobei das therapeutische Molekül aus der L-RNA besteht, insbesondere eine doppelsträngige L-RNA, beispielsweise ein Spiegelmer, ist.

6. Verwendung nach Anspruch 3, wobei das therapeutische Molekül ein mit der L-RNA covalent gebundenes Aptamer oder damit covalent gebundenen Antikörper enthält.

7. Verwendung nach einem der Ansprüche 3 und 5 bis 6, wobei die pharmazeutische Zusammensetzung das L-Ribozym in zumindest der Dosis enthält, welche der Dosis der Gabe der L-RNA entspricht, vorzugsweise in einer Dosis enthält, welche dem 2- bis 100-fachen, vorzugsweise dem 2- bis 20-fachen, der Dosis der Gabe der L-RNA entspricht.

8. Verwendung nach einem der Ansprüche 3 bis 7, wobei das L-Ribozym ein Hammerhead Ribozym ist.

9. Verwendung nach einem der Ansprüche 3 bis 8, wobei die pharmazeutische Zusammensetzung zusätzlich eine Nukleinsäure enthält, insbesondere ein 5- bis 20-mer, welches zur Aufschmelzung einer doppelsträngigen D-RNA oder L-RNA im Bereich der Targetsequenz befähigt ist.

10. Pharmazeutische Zusammensetzung enthaltend ein L-Ribozym zur Behandlung von unerwünschten physiologischen Nebenreaktionen, aufgrund der Gabe eines therapeutischen Moleküls enthaltend die L-RNA.

11. Pharmazeutische Zusammensetzung enthaltend ein L-Ribozym zur Behandlung oder Prophylaxe von Erkrankungen, welche mit einer Überexpression zumindest eines endogenen Genes einhergehen, wobei das L-Ribozym zur Spaltung einer Targetsequenz einer für das Gen codierenden endogenen Target-D-RNA befähigt ist

12. Verfahren zur Herstellung einer pharmazeutischen Zusammensetzung nach Anspruch 10 oder 11, wobei eine Sequenz aus L-Nukleotiden erstellt und synthetisiert wird, welche zur Spaltung einer vorgegebenen Sequenz aus L-Ribonukleotiden oder einer vorgegebenen Sequenz aus D-Ribonukleotiden befähigt ist, und wobei das L-Ribozym in pharmakologisch wirksamer Dosis zur Darreichung hergerichtet wird.

13. Verfahren nach Anspruch 12, wobei das L-Ribozym mit galenischen Hilfs- und/oder Trägerstoffen gemischt wird.

## Claims

1. The use of an L-ribozyme for producing a pharmaceutical composition.

2. The use according to claim 1, wherein the L-ribozyme is capable of splitting an L-RNA in the region of a target sequence of the L-RNA.

3. The use of an L-ribozyme, which is capable of splitting an L-RNA in the region of a target sequence of the L-RNA, for producing a pharmaceutical composition for treating undesired physiological adverse reactions due to the administration of a therapeutic molecule containing the L-RNA.

4. The use of an L-ribozyme for producing a pharmaceutical composition for the treatment or prophylaxis of diseases, which come along with an overexpression of at least of an endogenous gene, wherein the L-ribozyme is capable of splitting a target sequence of an endogenous target D-RNA coding for the gene.

5. The use according to claim 3, wherein the therapeutic molecule consists of the L-RNA, in particular is a double-stranded L-RNA, for instance a spiegelmer.

6. The use according to claim 3, wherein the therapeutic molecule comprises an aptamer covalently bound to the L-RNA or an antibody covalently bound thereto.

7. The use according to one of claims 3 and 5 to 6, wherein the pharmaceutical composition comprises the L-ribozyme at least at the dose, which corresponds to the dose of the administration of the L-RNA, preferably at a dose, which is 2 to 100 times, preferably 2 to 20 times the dose of the administration of the L-RNA.

8. The use according to one of claims 3 to 7, wherein the L-ribozyme is a hammerhead ribozyme.

9. The use according to one of claims 3 to 8, wherein the pharmaceutical composition additionally comprises a nucleic acid, in particular a 5- to 20-mer, which is capable of melting a double-stranded D-RNA or L-RNA in the region of the target sequence.

10. A pharmaceutical composition comprising an L-ribozyme for treating undesired physiological adverse reactions due to the administration of a therapeutic molecule containing the L-RNA.

11. A pharmaceutical composition comprising an L-ribozyme for the treatment or prophylaxis of diseases, which come along with an overexpression at least of an endogenous gene, wherein the L-ribozyme is capable of splitting a target sequence of an endogenous target D-RNA coding for the gene.

12. A method for producing a pharmaceutical composition according to claim 10 or 11, wherein a sequence of L-nucleotides is produced and synthesized, which is capable of splitting a predefined sequence of L-ribonucleotides or a predefined sequence of D-ribonucleotides, and wherein the L-ribozyme is prepared in a pharmacologically effective dose for the administration.

13. The method according to claim 12, wherein the L-ribozyme is mixed with galenic auxiliary and/or carrier substances.

## Revendications

1. Utilisation d'un L-ribozyme pour produire une composition pharmaceutique.

2. Utilisation selon la revendication 1, dans laquelle le L-ribozyme est apte à diviser un L-ARN dans la zone d'une séquence cible du L-ARN.

3. Utilisation d'un L-ribozyme, qui est apte à diviser un L-ARN dans la zone d'une séquence cible du L-ARN, pour produire une composition pharmaceutique destinée au traitement de réactions secondaires physiologiques indésirables à cause de l'administration d'une molécule thérapeutique contenant le L-ARN.

4. Utilisation d'un L-ribozyme pour produire une composition pharmaceutique pour le traitement et la prophylaxie de maladies, qui s'accompagnent d'une surexpression d'au moins un gène endogène, dans laquelle le L-ribozyme est apte à diviser une séquence cible d'un D-ARN cible endogène codant pour le gène.

5. Utilisation selon la revendication 3, dans laquelle la molécule thérapeutique consiste en le L-ARN, en particulier est un L-ARN bicaténaire, par exemple un spiegelmère.

6. Utilisation selon la revendication 3, dans laquelle la molécule thérapeutique comprend un aptamère lié par covalence au L-ARN ou un anticorps lié par covalence à celui-ci.

7. Utilisation selon une des revendications 3 et 5 à 6, dans laquelle la composition pharmaceutique comprend le L-ribozyme au moins à la dose, qui correspond à la dose de l'administration du L-ARN, de préférence à une dose, qui est 2 à 100 fois, de préférence 2 à 20 fois la dose de l'administration du L-ARN.

8. Utilisation selon une des revendications 3 à 7, dans laquelle le L-ribozyme est un ribozyme hammerhead.

9. Utilisation selon une des revendications 3 à 8, dans laquelle la composition pharmaceutique en addition comprend un acide nucléique, en particulier un 5- à 20-mère, qui est apte à fondre un D-ARN ou L-ARN bicaténaire dans la zone de la séquence cible.

10. Composition pharmaceutique comprenant un L-ribozyme destiné au traitement de réactions secondaires physiologiques indésirables à cause de l'administration d'une molécule thérapeutique contenant le L-ARN.

11. Composition pharmaceutique comprenant un L-ribozyme pour le traitement et la prophylaxie de maladies, qui s'accompagnent d'une surexpression d'au moins un gène endogène, dans laquelle le L-ribozyme est apte à diviser une séquence cible d'un D-ARN cible endogène codant pour le gène.

12. Procédé pour produire une composition pharmaceutique selon la revendication 10 ou 11, dans lequel une séquence de L-nucléotides est produite et synthétisée, qui est apte à diviser une séquence prescrite de L-ribonucléotides ou une séquence prescrite de D-ribonucléotides, et dans lequel le L-ribozyme est préparé dans une dose pharmacologiquement efficace pour l'administration.

13. Procédé selon la revendication 12, dans lequel le L-ribozyme est mélangé avec des substances auxiliaires et/ou porteurs galéniques.
